Europäisches Patentamt

(19) European Patent Office

Office européen des brevets

(11) Publication number : **0 478 327 A1**

# (12) EUROPEAN PATENT APPLICATION

(21) Application number : **91308792.0**

(22) Date of filing : **26.09.91**

(51) Int. Cl.⁵ : **A61K 7/32,** A61K 7/48, A61K 7/06

(30) Priority : **28.09.90 US 589362**

(43) Date of publication of application : **01.04.92 Bulletin 92/14**

(84) Designated Contracting States : **AT BE CH DE DK ES FR GB GR IT LI LU NL SE**

(71) Applicant : **CALGON CORPORATION Route 60-Campbell's Run Road Robinson Township Pennsylvania 15205 (US)**

(72) Inventor : **Melby, Allan L. 104 Heathercroft Drive Zelienople, PA 16063 (US)** Inventor : **Boothe, Jerry E. 221 Disney Drive Coraopolis, PA 15108 (US)**

(74) Representative : **Barrett-Major, Julie Diane et al Merck & Co., Inc. European Patent Department Terlings Park Eastwick Road Harlow Essex CM20 2QR (GB)**

(54) Method for thickening metal-containing products using DMDAAC/acrylic acid-type polymers.

㊄ A method for thickening metal-containing aqueous solutions, particularly personal care products, such as aluminum-containing antiperspirants, is disclosed. The method comprises adding an effective amount for the purpose of at least one dimethyl diallyl ammonium chloride/acrylic acid-type polymer.

EP 0 478 327 A1

## BACKGROUND OF THE INVENTION

The instant invention relates to the thickening of metal-containing aqueous solutions, such as personal care product formulations, particularly liquid antiperspirant formulations which contain aluminum, by the addition of an effective amount of at least one dimethyl diallyl ammonium chloride (DMDAAC)/acrylic acid-type polymer. These polymers increase the thickness or viscosity of aqueous metal-containing solutions such as personal care product formulations and the like, and generally do not impart color to water-white formulations being thickened.

Numerous references disclose cosmetic and personal care compositions, such as shampoos, antiperspirant formulations, anti-dandruff rinse conditioners, etc., which contain anionic and/or cationic polymers, an active agent, surfactants, emollients and other additives and preservatives commonly employed in the industry. Pertinent references which relate to DMDAAC polymers or which disclose cosmetic and personal care formulations containing DMDAAC polymers include:

1. U.S. Pat. No. 3,761,417, which is directed to detergent compositions containing particle deposition enhancing agents. More particularly, this patent discloses detergent and personal use detergent bars which contain water-soluble particles such as antimicrobial agents, surfactants and cationic polymers. Possible cationic polymers include dimethyl diallyl ammonium chloride polymers (DMDAAC).

2. U.S. Pat. No. 3,769,398, which discloses non-ionic hair shampoo formulations containing an active ingredient such as a betaine, sulfo betaine, amine oxide or mixture thereof, a water soluble polymer such as a polyethyleneimine-ethylene oxide or propylene oxide polymer or a propoxylated polyethyleneimine.

3. U.S. Pat. No. 4,329,335, which describes an amphoteric, nonionic anti-dandruff shampoo containing an active agent (1-imidazalyl-1-) (chlorophenoxy-3,3-dimethylbutane-2-one) and amphoteric surfactants. DMDAAC is disclosed as a preferred quaternized ammonium compound in this patent.

4. Published European Patent Application No. 74,819, which discloses an anti-dandruff cream rinse conditioner containing zinc pyrithione, glucan or guar gum, hydroxyethyl cellulose and a homopolymer of DMDAAC or a copolymer of DMDAAC and acrylamide.

5. U.S. Pat. No. 3,996,146, which discloses a shampoo formulation comprising from 0.05 to about 2.5%, by weight, of a cationic resin including quaternary polymers derived from dimethyl diallyl ammonium salts.

6. U.S. Pat. No. 4,040,984, which discloses polymers useful for preparing electroconductive paper which comprise quaternary diallyl dialkyl ammonium monomers and acrylic acid.

7. U.S. Pat. No. 3,912,808, which discloses a composition and method for waving or straightening hair using an aqueous solution of a reducing agent and a water soluble secondary or tertiary amine polymer or a polymer of diallyl amine or a quaternary polymer of diallyl dialkyl ammonium salts. This patent also discloses the use of dialkyl ammonium polymers which contain acrylamide or diacetone acrylamide. The use of dimethyl diallyl ammonium chloride/acrylic acid polymers is not disclosed or suggested.

8. U.S. Pat. No. 4,027,008, which discloses hair treating compositions which contain a water-soluble secondary or tertiary amine polymer or a polymer of diallyl amine or a quaternary polymer of diallyl dialkyl ammonium salts.

9. U.S. Pat. No. 3,986,825, which discloses the use of dialkyl diallyl ammonium polymers in cosmetic products, including copolymers of a dialkyl diallyl ammonium monomer and acrylamide or diacetone acrylamide.

10. U.S. Pat. No. 4,764,365, which discloses the use of DMDAAC/acrylic acid polymers such as Merquat 280 to improve the feel of personal care products. This patent discloses that commonly used antiperspirants include aluminum zirconium complex, aluminum chlorohydrate and the like. However, this patent does not disclose or suggest that DMDAAC/acrylic acid-type polymers may be used to thicken metal-containing aqueous solutions generally or aluminum-containing personal care products.

11. U.S. Pat. No. 4,772,462, which discloses the use of DMDAAC/acrylic acid-type polymers in shampoos, conditioners, rinses, bleaches, hair dyes and hair sprays.

12. U.S. Pat. No. 4,675,178, which relates to stable deodorant compositions which contain DMDAAC polymers and aluminum antiperspirant agents.

13. Copending application U.S.S.N. 794,981 discloses the use of DMDAAC/acrylic acid-type polymers in deodorant formulations.

In summary, though the use of dialkyl diallyl ammonium chloride-type polymers, including DMDAAC-acrylic acid type polymers, in personal care products is known, the use of polymers containing a diallyl dialkyl ammonium monomer and acrylic acid for the purpose of thickening aqueous solutions or personal care product compositions is not known or suggested in the art. These polymers are particularly effective when used to provide thickening to liquid personal care products, such as roll-on deodorant/antiperspirants, and generally do so without imparting color to water-white formulations.

## DETAILED DESCRIPTION OF THE INVENTION

The instant invention is directed to a method for thickening metal-containing aqueous compositions or solutions, such as liquid, water-based, metal-containing personal care product compositions or intermediates, comprising adding to such a composition or solution an effective amount of a polymer comprising:

a) about 1 to about 99%, based on total polymer weight, of a quaternary diallyl dialkyl ammonium monomer, wherein alkyl groups are independently selected from alkyl groups of 1 to 18 carbon atoms, preferably $C_{1-4}$ alkyl, and wherein said quaternary diallyl dialkyl ammonium monomer's counterion is selected from the group consisting of conjugate bases of acids having an ionization constant greater than $10^{-13}$, more preferably, selected from the group consisting of fluoride, bromide, chloride, hydroxide, nitrate, acetate, hydrogen, sulfate, and primary phosphates; and

b) about 1 to about 99%, based on total polymer weight, of an anionic monomer selected from the group consisting of acrylic acid and methacrylic acid; wherein the weight average molecular weight of said polymer ranges from about 5,000 to about 6,000,000, as determined by gel permeation chromatography.

The instant invention is also directed to thickened metal-containing aqueous compositions or solutions, such as thickened, personal care product compositions, which contain the above described polymer.

As used herein, the phrase "personal care product composition or solution" includes, but is not limited to, metal-containing hand and face lotions, soaps and creams, suntan lotions, bubble baths, shaving creams, antiperspirant and deodorant compositions or solutions.

As used herein, the term "metal-containing" refers to any aqueous composition or solution or personal care product formulation or product intermediate which contains a metal having a positive valence of greater than 2. Examples of metal ions of such metals include, but are not limited to, $Al^{+3}$, $Fe^{+3}$, $Cr^{+3}$ and $Ce^{-4}$. The most preferred metal is aluminum.

The most important class of aluminum-containing compositions are liquid personal skin care products, particularly roll-on antiperspirants. Such compositions are thickened by the instant polymers, as measured by the viscosity of the treated composition.

Personal skin care products generally comprise an active agent, such as a detergent, surfactant, conditioner, emollient, antimicrobial, antiperspirant and/or moisturizing agent. These products may also contain aluminum in the form of aluminum stearate, aluminum acetate, aluminum sulfate or other aluminum salt. Also, the active agents themselves may contain aluminum. For example, commonly used antiperspirants include, but are not limited to, aluminum zirconium complex, aluminum chlorohydrate and the like. For this reason, a preferred use of the instant polymer is for thickening water-based, liquid antiperspirant compositions, such as roll-on antiperspirants/deodorants. In the past, such compositions were generally thickened by the addition of emulsifying or dispersing fatty compounds, such as glycol stearates, cetyl alcohol or stearyl alcohol.

In its broadest sense, an effective amount of at least one of the instant polymers may be added to an aqueous solution, such as a liquid personal care product composition, in conjunction with a metal, such as aluminum, to thicken the solution being treated, if the solution does not contain a metal and the addition of a metal and at least one of the instant polymers does not cause compatibility or other problems.

As used herein, the term "effective amount" refers to that amount of polymer required to provide the desired thickness to the aqueous solution or aqueous-based product being treated. Generally, for compositions which contain a metal, the instant polymers are added at a dosage of greater than or equal to about 0.01% active polymer solids, based on the total weight of the liquid composition being treated. As the polymer dosage increases, assuming the presence of a suitable metal, the composition thickness as measured by viscosity generally increases from a water-like consistency to the consistency of a gel. Preferably, the dosage is about 0.01% to about 30% active polymer solids, based on the total weight of the metal-containing product to which the polymer is added. Most preferably, the polymer dosage should range from about 0.5% to about 12% active polymer, based on the total weight of the composition being thickened.

In the case where the aqueous solution or liquid personal care product to be thickened does not contain a metal having a positive valence of greater than 2, about 1.0 to about 50.0% of a suitable positive valence metal ion source, based on the total weight of the product being treated, can be added for the purpose. Any source of metal ions which is compatible with the solution, composition, intermediate or product to be thickened can be used. The amount of multivalent metal ions to be added varies depending upon the desired degree of thickening, the concentration of competing ions, if present, the anionic content of the polymer to be used and the specific metal ion added. In general, for a given polymer concentration, viscosity will increase with increasing metal ion concentration until the molar ratio of the metal ions to the total anionic functionality of the polymer (for example, the carboxylic functionality of an acrylic acid-based polymer), exceeds about one. Although the use of excess metal ions above this ratio is not harmful, it is not believed to cause a further thickening of a system being treated.

Further, ion solubility generally depends upon the pH of the aqueous system being treated. Also, the presence of other metal chelating or complexing anions, for example EDTA or NTA, can alter the ratio of metal ion to polymer necessary to obtain a designated thickness. The effective amount of metal ion is further influenced by the application pH, because the ratio of protonated to free carboxylate groups for a carboxylic group-containing polymer is a function of pH. The exact nature of this interaction depends on the disassociation constant of the carboxylic functional group, which varies with structure. For these reasons, the optimal metal ion to polymer ratio should be determined experimentally for each specific application. Notwithstanding the above considerations, the upper concentration of the metal ion source is oftentimes determined based on solubility considerations.

In addition to the metal ion source, an effective amount of at least one of the instant polymers is added, based on the desired thickness and the amount of metal ion source added. The instant polymers, and a metal ion source, if needed, can be added to aqueous solutions or compositions such as personal care product compositions by any convenient method. Order-of-addition is generally not critical. Mixing may be utilized to quickly disperse the polymer into the composition being treated.

The quaternary diallyl dialkyl ammonium monomer may comprise from about 1 to about 99%, based on total polymer weight, of the polymer while the anionic monomer may comprise from about 1 to about 99%, based on total polymer weight. Preferably, the quaternary: anionic weight ratio is from about 95:5 to about 50:50, and most preferably from about 95:5 to about 65:35, based on the total weight of polymer. Thus, in the polymers of the present invention, the cationic moiety of the polymer is preferably predominant while the anionic moiety of the polymer is preferably minor. Additionally, other moieties may be present in the instant polymers; for example, acrylamide or other nonionic moieties may be present.

An especially preferred polymer is that where the cationic portion is dimethyldiallyl ammonium chloride (DMDAAC) or diethyldiallyl ammonium chloride (DEDAAC) and where anionic portion is acrylic acid. Preferably, the DMDAAC/DEDAAC:acrylic acid weight ratio weight ratio ranges from about 95:5 to about 50:50, most preferably from about 95:5 to 65:35, based on total polymer weight.

The polymers of the instant invention may have any molecular weight ranging from about 5,000 to about 6,000,000, with the preferred molecular weight ranging from about 100,000 to about 5,000,000. The most preferred viscosity for the instant polymers ranges from about 4,000 to about 10,000 cps, as determined using a Brookfield LVF No. 4 spindle at 60 rpm. These polymers may be prepared using any conventional free radical polymerization technique, such as the technique disclosed by Butler and Angelo, "Journal of American Chemical Society," Vol. 79, p. 3128 (1957) or the technique suggested in U.S. Reissue Patent No. 28,543.

The most preferred polymers of the instant invention are commercially available copolymers of dimethyldiallyl ammonium chloride and acrylic acid which are sold as Merquat 280 by Calgon Corporation. Merquat 280 contains 80/20 (w/w) dimethyldiallyl ammonium chloride/acrylic acid and has a molecular weight of approximately 1 million.

Exemplary of the best mode is the use of Merquat 280 to thicken a water-based antiperspirant composition. Such compositions typically contain water, a glycol such as propylene glycol and an aluminum-containing antiperspirant. Because of the water-like consistency of such compositions, they generally must be thickened so that they do not flow too rapidly or easily past the balls of roll-on dispensers. An effective amount of Merquat 280 is added so as to yield the desired thickness.

Thus, in the best mode, an effective amount of Merquat 280 is added to a liquid aluminum-containing antiperspirant formulation capable of being topically administered wherein said formulation comprises water, an effective aluminum-containing antiperspirant, a glycol such as propylene glycol and optionally a deodorant and/or one or more cosmetically acceptable excipients such as buffers, fragrances and/or preservatives. The polymer is added in an amount sufficient to increase thickness to the desired consistency. The actual amount of polymer to be added depends primarily upon the desired thickness and the amount of aluminum in the composition being thickened. Generally, the preferred amount of Merquat 280 added will range from about 0.01% to about 30% active polymer, based on the total weight of the composition being thickened. Most preferably, the Merquat 280 dosage will range from about 0.5 to about 12% active polymer.

Typically a thickened antiperspirant formulation may be achieved by mixing at least one of the instant polymers with de-ionized water, followed by addition of the other ingredients. For example, an emulsifier may be added with agitation followed, in some instances, by heating to a temperature ranging from 65° to 90°C (preferably 75° to 80°). Agitation is then typically continued while adding at least one antimicrobial agent, followed by the addition of preservatives, buffers, dyes, and/or fragrances, and an antiperspirant agent.

The amount of antiperspirant agent used varies over a wide range and depends on the specific agent employed. Generally, an effective amount for the purpose is used. Representative antiperspirants within the scope of the invention include, but are not limited to, aluminum zirconium complex, aluminum chlorohydrate, aluminum chlorohydrex P.G. and the like.

Generally, the amount of polymer employed in the practice of the invention ranges from about 0.01% to about 30% polymer, by weight, based on the total weight of the formulation being treated. The remaining portion of the total composition contains the antiperspirant agent, water and conventional acceptable excipients.

Representative antimicrobial agents employed in the antiperspirant compositions of this invention include, but are not limited to, (2,4,4'-trichloro carbanilide), triclosan (2,4,4'-trichloro-2'-hydroxy diphenyl ether), benzalkonium chloride, zinc phenosulfonate, zinc ricinoleate and the like.

Representative emollients, humectants and moisturizing agents useful in such compositions include, but are not limited to, $C_{12-15}$ alcohol benzoates, sorbitol, glycerin, propylene glycols (PEG), lanolin, vegetable oils, mineral oils, isopropyl myristate, aloe vera, jojoba oil and the like.

Other cosmetically acceptable excipients that such formulations may contain include, but are not limited to, buffering agents and preservatives. Suitable water soluble preservatives are sodium bisulfate, sodium thiosulfate, ascorbate, benzalkonium chloride, glydant chlorobutanol, thimerosal, phenylmercuric borate, parabens, Tektamer[R] 38(1,2-dibromo-2,4-dicyanobutane) (Tektamer is a registered trademark of Calgon Corporation, Pittsburgh, PA.), benzyl alcohol, phenylethanol and the like. Suitable water soluble buffering agents are alkali or alkali earth carbonates, phosphates, bicarbonates, citrates, borates, acetates, acid anhydrides, succinates and the like, such as sodium phosphate, citrate, borate, acetate, bicarbonate and carbonate. These agents may be present in amounts sufficient to maintain some optimum pH of the system in the range 2 to 9. As such, the buffering agent can be as much as 20% on a weight basis of the total composition. Additional active agents are fully described in U.S. Pat. No. 3,986,825.

Generally, the amount of polymer employed in the practice of the invention ranges from about 0.01% to about 30% polymer, by weight, based on the total weight of the formulation being treated. The remaining portion of the total composition contains the antiperspirant agent, water and conventional acceptable excipients.

Known deodorant/antiperspirant manufacturing processes may require heat to bring conventional thickeners such as Cab-o-sil into solution. Such processes may be difficult to control and oftentimes result in a translucent or opaque products. By contrast, the instant method of thickening may generally be carried out at room temperature, is easy to control and generally results in a clear, water-white product.

EXAMPLES

The following examples, which further demonstrate the instant invention, are not intended to limit the scope of this invention in any way.

Examples 1-2

These examples demonstrate the use of the instant polymers in two (2) antiperspirant personal care product formulations:

|  | Example 1 | Example 2 |
|---|---|---|
| Merquat 280 | 1.0 to 10.0 | 1.0 to 3.5 |
| Water, D.I. | 24 - 33 | 51.5 - 54 |
| Propylene Glycol | 16.0 | 16.0 |
| Aluminum Chlorohydrate | 50 | - |
| Aluminum-Zirconium Chlorohydrate | - | 29.0 |

Examples 3-6

These examples demonstrate the ability of the instant polymers to increase the thickness of water-based aluminum-containing antiperspirant compositions.

EP 0 478 327 A1

|  | | Example No. | | |
|---|---|---|---|---|
|  | _3_ | _4_ | _5_ | _6_ |
| Merquat 280 Weight % | - | 3.5 | - | 2.0 |
| Deionized Water Weight % | 34.0 | 30.5 | 30.0 | 28.0 |
| Propylene Glycol Weight % | 16.0 | 16.0 | 16.0 | 16.0 |
| D.C. ACH 303 Aluminum Chlorhydrate Weight % | 50.0 | 50.0 | - | - |
| D.C. AZG 368 Aluminum Zirconium Chlorohydrate Glycine Weight % | - | - | 54.0 | 54.0 |
| Visc. (cps) | 11.5 | 25.0 | 6.8 | 23.5 |

In these examples, viscosity was measured by a Brookfield Viscometer, Model LVT, spindle #2 at 30 rpm.

**Claims**

1. A method for thickening a metal-containing, aqueous composition comprising adding to said composition an effective amount of a polymer comprising:
   a) about 1 to about 99%, by weight, based on the total weight of said polymer, of a quaternary diallyl dialkyl ammonium monomer, wherein alkyl groups are independently selected from alkyl groups of 1 to 18 carbon atoms and wherein said quaternary diallyl dialkyl ammonium monomer's counterion is selected from the group consisting of bases of acids having an ionization constant greater than $10^{-13}$,
   b) about 1 to about 99%, by weight, based on the total weight of said polymer, of an anionic monomer selected from the group consisting of acrylic acid and methacrylic acid; wherein the weight average molecular weight of said polymer ranges from about 5,000 to 6,000,000, as determined by gel permeation chromatography.

2. The method of Claim 1, wherein said alkyl group of a) is $C_{1-4}$.

3. The method of claim 1, wherein a) is selected from the group consisting of dimethyl diallyl ammonium chloride and diethyldiallyl ammonium chloride and b) is acrylic acid.

4. The method of claim 1, wherein said effective amount ranges from about 0.01 to about 20%, by weight, of said product.

5. The method of Claim 1, wherein said aqueous composition is a water-based personal care product which contains aluminum in the form of an aluminum stearate, aluminum acetate, aluminum sulfate, aluminum zirconium complex, aluminum chlorohydrate or aluminum chlorhydrex P.G.

6. The method of Claim 5, wherein said personal care product is a water-based antiperspirant and wherein said aluminum is in the form of aluminum zirconium complex, aluminum chlorohydrate or aluminum chlorhydrex P.G.

7. The method of Claim 1, wherein said counterion is selected from the group consisting of fluoride, chloride, bromide, hydroxide, nitrate, acetate, hydrogen sulfate and primary phosphates.

6

8. A thickened, water-based liquid antiperspirant which comprises:
   a) 1 to 95% water, by weight;
   b) 1 to 50% of aluminum-containing an antiperspirant, by weight; and
   c) an effective amount for thickening purposes of a copolymer comprising:
      1. about 1 to 99%, by weight of said polymer, of a monomer selected from the group consisting of dimethyl diallyl ammonium chloride and diethyldiallyl ammonium chloride; and
      2. about 1 to about 90%, by weight of said polymer, a monomer selected from the group consisting of acrylic acid, and methacrylic acid; wherein said polymer has a weight average molecular weight ranging from about 5,000 to about 6,000,000, as determined by gel permeation chromatography.

9. The composition of Claim 9, wherein said aluminum-containing antiperspirant compound is selected from the group consisting of aluminum zirconium complex, aluminum chlorohydrate, and aluminum chlorohydrex P.G.

10. A method for thickening an aqueous solution comprising adding to said solution an effective amount of:
   A) a polymer comprising:
      a) about 1 to about 99%, by weight, based on the total weight of said polymer, of a quaternary diallyl dialkyl ammonium monomer, wherein alkyl groups are independently selected from alkyl groups of 1 to 18 carbon atoms and wherein said quaternary diallyl dialkyl ammonium monomer's counterion is selected from the group consisting of bases of acids having an ionization constant greater than $10^{-13}$,
      b) about 1 to about 99%, by weight, based on the total weight of said polymer, of an anionic monomer selected from the group consisting of acrylic acid and methacrylic acid; wherein the weight average molecular weight of said polymer ranges from about 5,000 to 6,000,000, as determined by gel permeation chromatography.
   B) a metal ion having a positive valence of greater than 2, wherein the molar ratio of said metal ion to the carboxylic functionality of said polymer is greater than 1.

**European Patent Office**

## EUROPEAN SEARCH REPORT

Application Number

EP 91 30 8792

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | EP-A-0 266 111 (CALGON CORP.) <br> * Whole document * & US-A-4 764 365 <br> --- | 1-10 | A 61 K 7/32 <br> A 61 K 7/48 <br> A 61 K 7/06 |
| D,A | EP-A-0 200 548 (CALGON CORP.) <br> * Whole document * & US-A-4 675 178 <br> --- | 1-10 | |
| D,A | EP-A-0 269 243 (CALGON CORP.) <br> * Claims * & US-A-4 772 462 <br> --- | 1-3 | |
| D,A | EP-A-0 074 819 (AMWAY CORP.) <br> * Claim 15 * <br> ----- | 1-4 | |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 17-12-1991 | COUCKUYT P.J.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)